(19) Europäisches Patentamt
European Patent Office
Office européen des brevets

(11) Veröffentlichungsnummer: **0 036 135**
**A2**

(12) # EUROPÄISCHE PATENTANMELDUNG

(21) Anmeldenummer: **81101571.8**

(51) Int. Cl.³: **A 61 K 7/32**

(22) Anmeldetag: **05.03.81**

(30) Priorität: **13.03.80 DE 3009542**

(43) Veröffentlichungstag der Anmeldung:
**23.09.81 Patentblatt 81/38**

(84) Benannte Vertragsstaaten:
**AT BE CH DE FR GB IT LI LU NL SE**

(71) Anmelder: **Henkel Kommanditgesellschaft auf Aktien
-Patentabteilung- Postfach 1100 Henkelstrasse 67
D-4000 Düsseldorf 1(DE)**

(72) Erfinder: **Möller, Heinrich, Dr.
Erlanger Strasse 45
D-4000 Düsseldorf 13(DE)**

(72) Erfinder: **Osberghaus, Rainer, Dr.
Südallee 47
D-4000 Düsseldorf 13(DE)**

(54) **Desodorierende kosmetische Zusammensetzungen.**

(57) Gegenstand der Erfindung sind desodorierende kosmetische Zusammensetzungen mit einem Gehalt an Aminobenzoesäure-amiden der Formel

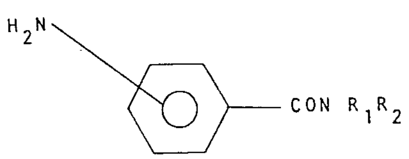

in der $R_1, R_2$ = H, Alkylrest mit 1 - 12 C-Atomen,
Hydroxyalkylrest mit 2 - 4 C-Atomen,
Aryl-, Aralkylrest oder
Carboxyalkylrest mit 1 - 3 C-Atomen,
zusammen mit N ein Heterocyclus.

Die desodorierenden Zusammensetzungen enthalten bevorzugt 0,1 - 5 Gewichtsprozent der Aminobenzoesäureamide und 0,01 - 1 Gewichtsprozent Antioxidantien neben sonst für desodorierende Zubereitungen üblichen Komponenten.

EP 0 036 135 A2

# Patentanmeldung

D 6166 EP

"Desodorierende kosmetische Zusammensetzungen"

Die Erfindung betrifft desodorierende kosmetische Zusammensetzungen mit einem Gehalt an Amino-benzoesäureamiden.

Es ist bekannt, daß der störende Geruch, der die Perspiration des Menschen begleitet, durch die bakterielle Zersetzung des zunächst geruchlosen Schweißes verursacht wird. Es hat daher nicht an Vorschlägen gefehlt, diesem Übelstand zu begegnen, ohne daß es bisher zu einer allseits befriedigenden Lösung gekommen wäre. Im wesentlichen handelt es sich um zwei Wege, die zur Lösung des Problems eingeschlagen wurden, einmal um den Einsatz antimikrobieller Verbindungen zur Abtötung beziehungsweise Wachstumshemmung der bakteriellen Hautflora, die die Zersetzung des Schweißes verursacht, zum anderen um den Einsatz von Verbindungen, die die Schweißabsonderung unterbinden. Daneben spielen noch rein sorptiv wirkende sowie geruchsüberdeckende Mittel eine völlig untergeordnete Rolle. Sowohl der Einsatz der die Schweißabsonderung unterbindenden Antitranspirantien als auch der antimikrobiellen Verbindungen ist umstritten, da im ersten Fall ein Eingriff in den physiologischen Vorgang der Schweißbildung erfolgt, was unerwünscht ist und im zweiten Fall neben der Wachstumshemmung der die Schweißzersetzung bewirkenden Bakterien auch ein Eingriff in die natürliche Hautflora erfolgt. Darüber hinaus kann der längere Gebrauch von Antitranspirantien zu Hautreizungen und zu Hautveränderungen führen. Bei den kosmetischen Mitteln mit desodorierender Wirkung handelt es sich durchweg um

Mittel mit einem Gehalt an antimikrobiellen Stoffen. Als solche werden zum Beispiel Phenolderivate mit und ohne Halogensubstituenten, quartäre Ammoniumverbindungen, desinfizierend wirkende Abkömmlinge von Aminosäuren vorgeschlagen. Wenn bei dem Einsatz der Deodorantien die Gefahr von Hautreizungen nicht in so hohem Maße wie bei der Verwendung von Antitranspirantien heraufbeschworen wird, so treten auch bei der laufenden Benutzung von Antimikrobika enthaltenden Deodorantien neben der Schädigung der hauteigenen Flora gelegentliche Unverträglichkeiten, Lichtsensibilisierungen und toxische Nebenwirkungen unterschiedlicher Stärke auf. Darüber hinaus ist die Mehrzahl dieser Produkte nicht geruchlos, manche besitzen einen leicht phenolischen Geruch, der bei vielen Benutzern auf Ablehnung stößt. Man ist daher weiterhin bestrebt, sehr gut desodorierende, von Nebenwirkungen weitgehend freie kosmetische Mittel zur Unterdrückung von Körpergeruch herzustellen.

Es wurde nun gefunden, daß desodorierende kosmetische Zusammensetzungen mit einem Gehalt an Amino-benzoesäureamiden der allgemeinen Formel

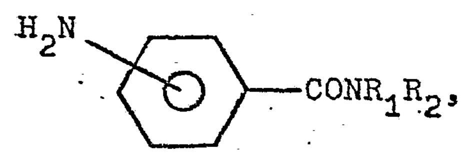

in der $R_1$ und $R_2$ unabhängig voneinander für Wasserstoff, einen Alkylrest mit 1 - 12 Kohlenstoffatomen, einen Hydroxyalkylrest mit 2-4 Kohlenstoffatomen, einen Aryl-, Aralkyl- oder Carboxyalkylrest mit 1 - 3 Kohlenstoffatomen stehen, beziehungsweise zusammen mit dem Amidstickstoff einen gegebenenfalls substituierten heterocyclischen Ring bilden, die gestellten Anforderungen weitgehend erfüllen.

/3

Die Herstellung der erfindungsgemäß einzusetzenden Amino-benzoesäureamide erfolgt nach allgemein bekannten Verfahren der organischen Synthese. So lassen sich zunächst die Nitrobenzamide durch Aminolyse der Nitrobenzoylchloride und daraus durch katalytische Hydrierung die entsprechenden Amino-benzoesäureamide herstellen.

Als den erfindungsgemäß einzusetzenden Amino-benzoesäureamiden zugrundeliegende Carbonsäuren sind die ortho-, meta- und para-Aminobenzoesäure anzuführen.

Als Amidkomponente kommen neben primären Amiden z.B. Methyl-, Dimethyl-, Ethyl-, Diethyl-, Propyl-, Dipropyl-, Methylpropyl-, Di-2-propyl-, Butyl-, Dibutyl-, sek. Butyl-, tert. Butyl-, Hexyl-, Dihexyl-, 2-Ethylhexyl-, Octyl-, Decyl-, Dodecyl-, Ethanol-, Diisopropanol-, 3-Methoxypropyl-, 3-(2-Ethylhexoxy)-propyl-, Benzyl-amid, Anilid, N-Methylanilid, Piperidid, 2-Methyl-, 3-Methyl-, 4-Methyl-, 2,6-Dimethyl-, 3,5-Dimethyl-piperidid, Morpholid, 2,6-Dimethylmorpholid in Frage.

Als erfindungsgemäß einzusetzende Amino-benzoesäureamide sind z. B. p-Amino-benzoesäure-diethylamid, -dipropylamid, -anilid, -piperidid, -4-methylpiperidid, -3-methylpiperidid, -carboxymethylamid zu nennen.

/4

Zur Herstellung der erfindungsgemäßen desodorierenden kosmetischen Zusammensetzungen können die Amino-benzoesäureamide in alle üblicherweise für Deodorantien gebräuchlichen Zubereitungen eingearbeitet werden wie Puder, Stifte, Roll-on und Sprays, wobei der Deo-Spray das bevorzugte Einsatzgebiet ist. Die Einarbeitung erfolgt in bekannter Weise durch einfaches Vermischen oder Lösen in den anderen Komponenten der Zubereitung wie Lösungsmitteln, Wachsen, Fettsübstanzen, Polyglykolen, Pudergrundstoffen. Die erfindungsgemäßen desodorierenden kosmetischen Zusammensetzungen enthalten dabei die Amino-benzoesäureamide in Mengen von 0,1 bis 5 Gewichtsprozent, vorzugsweise 0,5 bis 2 Gewichtsprozent, bezogen auf die gesamte Zusammensetzung.

Die erfindungsgemäßen desodorierenden kosmetischen Zusammensetzungen werden die Amino-benzoesäureamide bevorzugt als alleinige Deo-Wirkstoffe enthalten, jedoch ist auch eine Kombination mit anderen desodorierenden Wirkstoffen möglich.

Die desodorierende Wirkung der erfindungsgemäßen kosmetischen Zusammensetzungen läßt sich steigern, wenn diese neben den Amino-benzoesäureamiden Antioxidantien in Mengen von 0,01 bis 1 Gewichtsprozent, vorzugsweise 0,05 bis 0,5 Gewichtsprozent, bezogen auf die gesamte Zubereitung enthalten.

Als in den erfindungsgemäßen Zusammensetzungen einzusetzende Antioxidantien sind alle auf dem pharmazeutischen, kosmetischen und Nahrungsmittel-Sektor gebräuchlichen Antioxidantien geeignet und es sind folgende Produkte zu nennen:

Butylhydroxytoluol, Butylhydroxyanisol, Guajakharz, Lecithin, Nordihydroguajaretsäure, Propylgallat, Octylgallat, Dodecylgallat, Tocopherole, Trihydroxybutyrophenon, Ascorbinsäure, Ascorbylpalimitat, Dilaurylthiodipropionat, Distearylthiodipropionat, Monoisopropylcitrat, Thiodipropionsäure und Citraconsäure.

Die nachfolgenden Beispiele sollen den Gegenstand der Erfindung näher erläutern, ohne ihn jedoch hierauf zu beschränken.

/6

## Beispiele

Zunächst werden einige der in den erfindungsgemäßen desodorierenden kosmetischen Zusammensetzungen einzusetzenden Amino-benzoesäureamide näher beschrieben.

Produkt A. p-Amino-benzoesäure-dipropylamid.

a) p-Nitro-benzoesäure-dipropylamid.
Zu einer Lösung von 50,6 g (0,5 Mol) Dipropylamin in 400 ml Ether wurden unter Rühren bei Raumtemperatur 37,1 g (0,2 Mol) p-Nitro-benzoylchlorid in 300 ml Ether getropft. Anschließend wurde 1 Stunde lang bei Raumtemperatur und 1 Stunde lang bei Siedetemperatur gerührt, das Reaktionsgemisch mit verdünnter Salzsäure behandelt, die ätherische Phase mit Wasser gewaschen und eingedampft. Es wurden 44,3 g p-Nitro-benzoesäure-dipropylamid als hellgelber, öliger Rückstand erhalten.

b) p-Amino-benzoesäure-dipropylamid.
44,3 g (0,2 Mol) des p-Nitro-benzoesäure-dipropylamids wurden in 500 ml Ethanol gelöst und nach Zugabe von 5 g Palladium/Aktivkohle (5 %ig) bei 50° C und 60 bar hydriert. Nach Filtrieren, Eindampfen der Ethanollösung und Umkristallisieren des Rückstandes (34,1 g) aus Ether wurden 26,7 g p-Amino-benzoesäure-dipropylamid vom Schmelzpunkt 78-79° C erhalten.

In analoger Weise wurden die folgenden Verbindungen hergestellt.

/7

Produkt B: p-Amino-benzoesäure-anilid, Schmp. $130^{\circ}$ C.

Produkt C: p-Amino-benzoesäure-diethylamid, Schmp. $122-123^{\circ}$C.

Produkt D: p-Amino-benzoesäure-piperidid, Schmp. $160-161^{\circ}$C.

Produkt E: p-Amino-benzoesäure-4-methylpiperidid, Schmp. $114-115^{\circ}$ C.

Produkt F: p-Amino-benzoesäure-3-methylpiperidid, Schmp. $155^{\circ}$ C.

Produkt G: p-Amino-benzoesäure-carboxymethylamid, Schmp. $199-200^{\circ}$ C.

/8

Nachstehend werden einige Beispiele für erfindungsgemäße desodorierende kosmetische Zusammensetzungen angegeben.

Desodorierender Stift

| | | |
|---|---|---|
| 2-Octyldodecanol | 27,0 | Gewichtsteile |
| Cetylstearylalkohol | 4,0 | " |
| Natriumstearat | 9,0 | " |
| Kokosfettsäuremonoethanolamid | 3,0 | " |
| Paraffinöl | 4,0 | " |
| Propylenglykol | 2,0 | " |
| Ethanol | 49,0 | " |
| Produkt A | 2,0 | " |

Desodorierender Puder

| | | |
|---|---|---|
| Reisstärke | 27,0 | Gewichtsteile |
| Magnesiumcarbonat | 3,0 | " |
| Zinkoxid | 2,0 | " |
| Talkum extra fein | 81,0 | " |
| Produkt B | 2,0 | " |

Desodorierender Spray

| | | |
|---|---|---|
| Produkt D | 1,5 | Gewichtsteile |
| Ethanol | 11,0 | " |
| Isopropanol | 20,5 | " |
| Isopropylmyristat | 2,0 | " |
| Treibgas Frigen 12/114 60:40 | 65,0 | " |

Desodorierender Spray

| | | |
|---|---|---|
| Produkt E | 1,3 | Gewichtsteile |
| Butylhydroxytoluol | 0,2 | " |
| Propylenglykol | 2,5 | " |
| Isopropylmyristat | 2,0 | " |
| Ethanol | 14,0 | " |
| Treibgas Frigen 11/12 50:50 | 80,0 | " |

Deodorant-Creme

| | | |
|---|---|---|
| Gemisch von Mono- und Diglyceriden der Palmitin- und Stearinsäure, Cutina MD(R) Dehydag | 20,5 | Gewichtsteile |
| Cetyl/Stearylalkohol + ca. 12 Mol Ethylenoxid Eumulgin B1(R) Dehydag | 2,0 | " |
| Produkt G | 1,3 | " |
| Butylhydroxytoluol | 0,2 | " |
| Wasser | 61,9 | " |
| p-Hydroxybenzoesäuremethylester | 0,1 | " |
| Parfümöl | 1,0 | " |

Deodorans für Pumpzerstäuber

| | | |
|---|---|---|
| Ethanol | 85,0 | Gewichtsteile |
| Isopropanol | 7,2 | " |
| Produkt F | 1,5 | " |
| Butylhydroxytoluol | 0,3 | " |
| Parfüm | 1,0 | " |
| Wasser | 5,0 | " |

Für eine vergleichende Wirksamkeitsprüfung wurde der folgende desodorierende Spray hergestellt.

Desodorierender Spray A

| | | |
|---|---|---|
| Produkt C | 1,5 | Gewichtsteile |
| Isopropanol | 5,5 | " |
| Ethanol | 33,0 | " |
| Treibgas Frigen 12/114 60:40 | 60,0 | " |

/10

Vergleichsspray B

| | | |
|---|---|---|
| Cypryl-/Caprinsäuretriglycerid | 1,5 | Gewichtsteile |
| Isopropanol | 5,5 | " |
| Ethanol | 33,0 | " |
| Treibgas Frigen 12/114 60:40 | 60,0 | " |

Eine Testgruppe, bestehend aus 15 weiblichen und 15 männlichen Teilnehmern, benutzte zunächst 5 Tage lang eine von antimikrobiellen Mitteln freie Seife F und keine Deodorantien oder Antiperspirantien. Nach dieser Zeit erhielt jeder Teilnehmer ein T-Shirt und die Instruktion, am 6. Tage morgens nach der Wäsche mit der Seife F eine Achsel mit dem Deo-Spray A zu behandeln und die andere Achsel zum Vergleich nicht zu behandeln, wobei die eine Hälfte der Gruppe die linke Achsel, die andere Hälfte die rechte Achsel behandelte. Die Geruchsentwicklung wurde von den Versuchspersonen selbst sowie von zwei erfahrenen Kosmetikern durch Abriechen der T-Shirts nach 8 Stunden und 24 Stunden beurteilt. Hiernach wurde von den Versuchspersonen eine Woche lang lediglich die Seife F benutzt.Anschließend wurde der Test wiederholt, wobei die zuvor unbehandelte Achsel mit dem Deo-Spray A behandelt wurde und die andere Achsel zum Vergleich diente.

In beiden Tests wurde von allen am Versuch beteiligten Personen eine sehr gute Geruchsunterbindung durch den desodorierenden Spray A festgestellt.

Mit der gleichen Testgruppe wurde der Test in völlig analoger Form wiederholt, nur daß an Stelle des Deo-Sprays A jeweils der Vergleichsspray B eingesetzt wurde.

Bei diesem Versuch konnte von keiner der am Versuch beteiligten Personen eine signifikante Geruchsminderung festgestellt werden.

/11

Patentansprüche:

1. Desodorierende kosmetische Zusammensetzungen, gekennzeichnet durch einen Gehalt an Amino-benzoesäureamiden der allgemeinen Formel

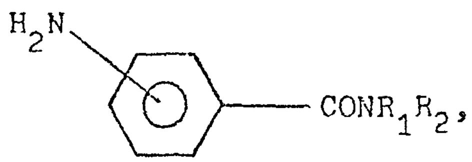

in der $R_1$ und $R_2$ unabhängig voneinander für Wasserstoff, einen Alkylrest mit 1- 12 Kohlenstoffatomen, einen Hydroxyalkylrest mit 2 - 4 Kohlenstoffatomen, einen Alkoxyalkylrest mit 1 - 8 Kohlenstoffatomen im Alkoxyrest und 1 - 3 Kohlenstoffatomen im Alkylrest, einen Aryl-, Aralkyl- oder Carboxyalkylrest mit 1 - 3 Kohlenstoffatomen stehen, beziehungsweise zusammen mit dem Amidstickstoff einen gegebenenfalls substituierten heterocyclischen Ring bilden.

2. Desodorierende kosmetische Zusammensetzungen nach Anspruch 1, dadurch gekennzeichnet, daß sie die Aminobenzoesäureamide in einer Menge von 0,1 bis 5 Gewichtsprozent, vorzugsweise 0,5 bis 2 Gewichtsprozent, bezogen auf die gesamte Zusammensetzung, enthalten.

3. Desodorierende kosmetische Zusammensetzungen nach Anspruch 1 und 2, dadurch gekennzeichnet, daß sie neben den Aminobenzoesäureamiden Antioxidantien in einer Mange von 0,01 bis 1 Gewichtsprozent, vorzugsweise 0,05 bis 0,5 Gewichtsprozent, bezogen auf die gesamte Zubereitung, enthalten.